Europäisches Patentamt

European Patent Office

Office européen des brevets

(11) Veröffentlichungsnummer: **0 401 594**
**A2**

# (12) EUROPÄISCHE PATENTANMELDUNG

(21) Anmeldenummer: **90109751.9**

(22) Anmeldetag: **22.05.90**

(51) Int. Cl.$^5$: **A61L 2/18, A61B 1/12**

(30) Priorität: **06.06.89 DE 3918432**

(43) Veröffentlichungstag der Anmeldung:
**12.12.90 Patentblatt 90/50**

(84) Benannte Vertragsstaaten:
**BE DE FR GB NL**

(71) Anmelder: **RIWOPLAN MEDIZIN-TECHNISCHE EINRICHTUNGSGESELLSCHAFT MBH**
**Pforzheimer Strasse 24**
**D-7134 Knittlingen(DE)**

(72) Erfinder: **Mönch, Harry**

Schwabstrassee 4
**D-7134 Knittlingen(DE)**
Erfinder: **Weisert, Willi**
**Mühlweg 11**
**D-7519 Oberderdingen(DE)**
Erfinder: **Rentschler, Gunter**
**Obere Hofstadt 42**
**D-7527 Kraichtal-Münzesheim(DE)**

(74) Vertreter: **Wilcken, Thomas, Dipl.-Ing. et al**
**Musterbahn 1**
**D-2400 Lübeck(DE)**

(54) Verfahren zum Desinfizieren von Endoskopen und Sterilisator zur Durchführung des Verfahrens.

(57) Das vorgeschlagene Verfahren zum Desinfizieren von flexiblen Endoskopen in einer mit Spül-, Desinfektions- und Neutralisationsflüssigkeit (9,10) beschickbaren, in einem fahrbaren Gehäuse (2) angeordneten Wanne (7) kennzeichnet sich dadurch, daß für ein in die Wanne eingelegtes Endoskop ein Desinfektionsprogramm aus Reinigen des äußeren Endoskopschaftes mittels strömenden, manuell in die Wanne (7) zu füllenden Spülwassers unter Zugabe eines flüssigen Spülmittels, aus Prüfung der Endoskopkanäle (1,2,3) auf Durchgängigkeit mittels der im Kreislauf geführten Spülflüssigkeit, aus Ablauf der Spülflüssigkeit, aus Durchblasen der Kanäle, aus Einfüllen eines flüssigen Desinfektionsmittels (9) in die Wanne (7) mit deren Kreislauf durch die Kanäle, aus Ablauf des flüssigen Desinfektionsmittels mit nachfolgendem Durchblasen der Kanäle und aus Neutralisation des in den Kanälen befindlichen restlichen Desinfektionsmittels (9) durch das ein Sterilfilter (6) durchströmende Wasser mit anschließendem Durchblasen durchgeführt wird. Jedem Einzelschritt des Desinfektionsprogrammes geht eine entsprechende, auf einem ablesbaren Anzeigenfeld (17) in Klartext erscheinende Anzeige in Form einer Anweisung sowie ein visuelles und akustisches Signal voraus, aufgrund dessen dann der von Hand auszulösende Desinfektionsvorgang des netzunabhängig zu betreibenden Desinfektors auszuführen ist

FIG. 7

EP 0 401 594 A2

Die Erfindung geht von einem Desinfektor zum Desinfizieren von Endoskopen aus, bei dem eine Wanne in einem fahrbaren Gehäuse ein Endoskop aufnimmt, ·wobei die Wanne mit Spül-, Desinfektions- und Neutralisationsflüssigkeit beschickbar ist.

Bekannte Desinfektoren haben den Nachteil, daß das Endoskop während der verschiedenen Vorgänge beim Desinfizieren von einer in eine zweite Wanne umgesetzt werden muß, so daß dadurch eine Kontamination des bereits desinfizierten Endoskopes eintreten kann.

Es ist auch bekannt, eine Reinigungs- und Desinfektionswanne in einem fahrbaren Gehäuse anzuordnen, wobei aber ebenfalls Kontaminationen eintreten können, da einzelne Aufnahmegefäße für mehrere aufeinanderfolgende Arbeitsgänge Verwendung finden.

Die Erfindung geht nun von halbautomatisch arbeitenden Verfahren und Desinfektoren der erwähnten Art aus und hat zur Aufgabe, daß die aufeinanderfolgenden Programmschritte für die Desinfektion von Endoskopen selbst für einen Laien ohne weiteres von Hand durchgeführt werden können, ohne daß eine Kontamination eintreten kann.

Diese Aufgabe wird durch das Verfahren nach dem Anspruch 1 und den Desinfektor nach dem Anspruch 2 gelöst. Vorteilhafte Weiterbildungen ergeben sich aus den Unteransprüchen.

Durch das erfindungsgemäße Verfahren und den erfindungsgemäßen Desinfektor für Endoskope werden die aufeinanderfolgenden Programmschritte jeweils durch die Elektronik auf einem Anzeigenfeld angezeigt und können sodann durch Handbetätigung durch eine beliebige Bedienung ausgelöst werden, wobei in keinem Falle Verschmutzungen und Kontaminationen eintreten können.

Die jeweils zu betätigende Taste leuchtet auf bzw. die Richtung (Betätigungsrichtung) des Umschalthebels wird angezeigt. Eine Betätigung von falschen oder mehreren Tasten gleichzeitig wird von der Elektronik verhindert.

Die Erfindung mit weiteren vorteilhaften Merkmalen wird nachstehend anhand der Zeichnungen beschrieben und näher erläutert:

Es zeigt:

Figur 1 : den erfindungsgemäß ausgebildeten Desinfektor in der Frontansicht;

Figur 2 : den Desinfektor nach Figur 1 in einer ersten Seitenansicht;

Figur 3 : den Desinfektor nach Figur 1 in der Rückseitenansicht;

Figur 4 : den Desinfektor in der zweiten Seitenansicht mit entfernter seitlicher Gehäuseabdeckung und Einblick in das Geräteinnere;

Figur 5 : den Desinfektor nach Figur 1 in einer Draufsicht;

Figur 6 : die Aufsicht auf den vorderen Teil der Figur mit dem Tasten- und Anzeigenfeld zur Bedienung des erfindungsgemäßen Desinfektors in vergrößerter Darstellung;

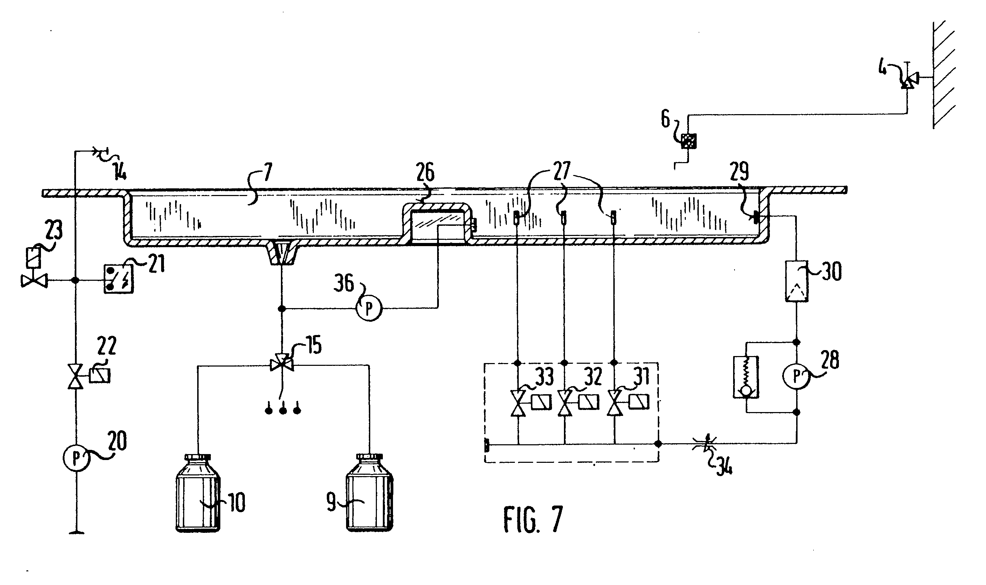

Figur 7 : das Funktionsschema des Desinfektors nach der Erfindung.

Der Desinfektor besteht im wesentlichen aus einem fahrbaren Gehäuse 2, das über eine Schlauchleitung 3 mit dem Wasseranschluß 4 verbunden ist. Das dem Wasseranschluß 4 abgekehrte Schlauchende ist über eine Steigleitung mit einem schwenkbeweglich angeordneten Wasserhahn 5 verbunden, so daß die Wasserzufuhr geregelt bzw. abgesperrt werden kann. An den Hahn 5 schließt sich ein Sterilfilter 6 an, dessen unterer Auslaß direkt in die das Endoskop aufnehmende Wanne 7 mündet, die von oben zum Einlegen eines Endoskopes nach Entfernen einer transparenten Abdeckung 8, frei zugänglich ist. Die Abdeckung 8 weist eine Ausnehmung 9 auf, die der untere Gehäuseteil des Filters 6 durchgreift, um während der Desinfektion eines Endoskopes die Abdeckung 8 nicht entfernen zu müssen.

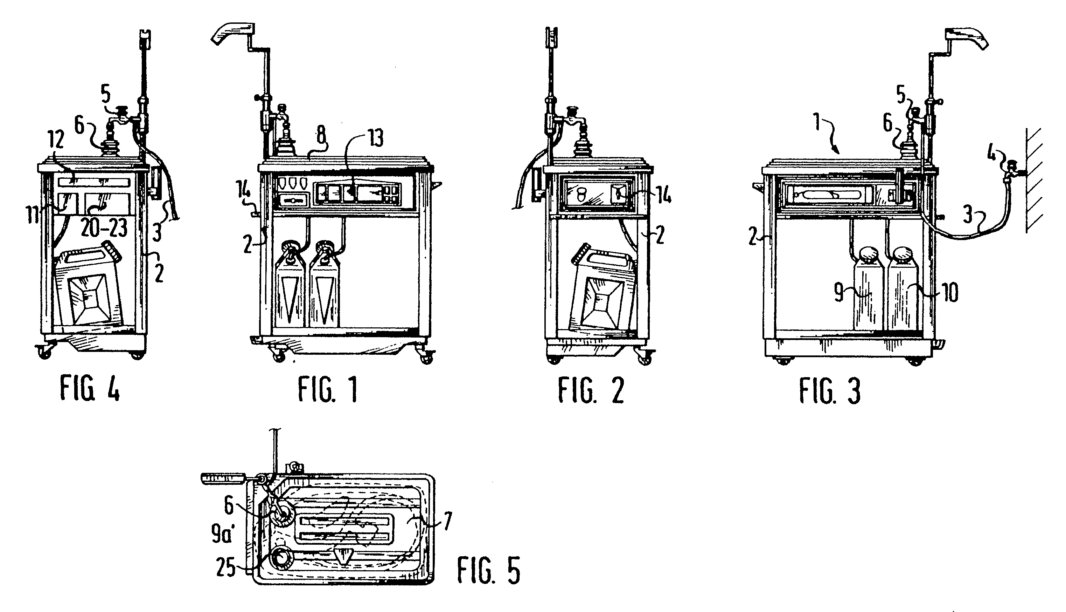

In dem fahrbaren Gehäuse sind die die Desinfektionsflüssigkeit 9 und die Neutralisationsflüssigkeit 10 aufnehmenden Behälter (Figur 3) sowie ein Akkumulator 11 und auch die Steuerelektronik 12 nach Figur 4 angeordnet.

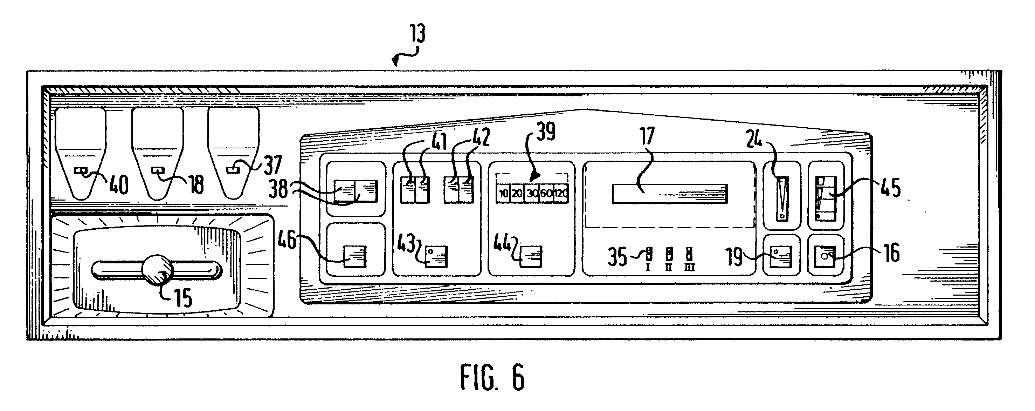

Im oberen Teil des Gehäuses 2 befindet sich an dessen Frontseite das Tastenfeld 13 der Steuerelektronik, das entsprechend der Figur 6 aufgeteilt ist. Um nun nach eingelegtem Endoskop den Programmablauf zu starten, ist es zunächst erforderlich, den am Endoskopsteuergehäuse befindlichen Schlauchanschlußstutzen über eine Schlauchverbindung mit dem Anschluß 14 zu verbinden. Im Anschluß daran werden alle Kanäle des Endoskopes mit den in der Wanne 7 befindlichen Anschlußstutzen 27 verbunden.

Nach dem Anschließen der Endoskopkanäle ist zu Beginn des Desinfektionsablaufes zunächst der Umschalthebel 15 in Mittelstellung "Betrieb" zu bringen und der Taster 16 zu betätigen, um den Desinfektor 1 einzuschalten. Auf dem Display 17 erscheint nun die Anzeige "Programm-Ende", die Anzeige 18 "Betrieb" leuchtet auf und die "Start-Taste" 19 beginnt zu blinken. Dieses Blinksignal ist ein Hinweis für die Bedienungsperson, diese Taste zu betätigen, um dadurch die Überprüfung des Endoskopschaftes auf Dichtigkeit hin einzu leiten. Mittels der Pumpe 20 wird nun Luft, die aus der Atmosphäre entnommen wird, über den Anschluß 14 und über die mit dem Endoskop am Steuergehäuse gekuppelte Schlauchleitung in den Endoskopinnenraum geleitet, um dadurch einen geringen Überdruck im Endoskopinneren zu erzeugen, der während des gesamten Desinfektionsablaufes aufrechterhalten wird. Um einen ständigen Betrieb

2

der Druckpumpe 20 zu vermeiden, wird, nach Erreichen des Überdrucksollwertes im Innenraum des Endoskopes, der Strömungsweg zwischen der Pumpe 20 und dem Anschlußstutzen 14 mittels des Magnetventils 22 und die Entlüftung über das Magnetventil 23 durch die jeweils vorerwähnten Magnetventile verschlossen.

Sollte bei der Überprüfung eine Undichtigkeit anhand eines mit dem Druckmesser 21 zu messenden Druckverlustes festgestellt werden, so erscheint auf dem Display 17 der Hinweis "Endoskop undicht". In diesem Fall wird durch die Elektronik 12 der Beginn des Desinfektionsablaufes abgebrochen und ein Neustart für eine bestimmte Zeit verhindert. Ein Wiedereinschalten des Desinfektors ohne Entfernung des als schadhaft erkannten Endoskopes ist nicht möglich, da mit Beginn der Dichtigkeitsüberprüfung der im Endoskopinnenraum zu erzeugende Überdrucksollwert nicht aufgebaut werden kann und damit der Neustart durch die Elektronik verhindert wird. Gleichzeitig mit dem Hinweis auf dem Display 17 ertönt ein Signal und das Anzeigenfeld 24 "Fiberskop undicht" leuchtet als Zusatzinformation auf.

Kann im Gegensatz zum zuvor geschilderten Fall eine Undichtigkeit nicht festgestellt werden, so erscheint auf dem Display 17 die Anzeige "Wasser einfüllen". Die Zuführung von Wasser erfolgt durch manuelles Betätigen des Wasserhahnes 5. Gleichzeitig mit dem Einfüllen von Wasser durch die Öffnung 9 in der tranparenten Abdeckung 8 kann durch die verschließbare Ötfnung 25 ein Zusatzmittel, für die äußere Reinigung des Endoskopes, zugegeben werden. Ist die Wanne 7 bis zur Höhe des Steges 26 mit Wasser gefüllt, so ist die Wasserzufuhr zu beenden und wiederum die blinkende Taste 19 zu betätigen, um den Vorgang der Überprüfung der Kanäle 1 bis 3, denen die Anschlüsse 27 in der Wanne 7 zugeordnet sind, einzuleiten. Diese Überprüfung erfolgt dadurch, daß mittels der Saug- und Druckpumpe 28 über die Öffnung 29, das Schmutzfilter 30 und die Magnetventile 31 bis 33 die Spülflüssigkeit durch die Endoskopkanäle hindurchgespült wird. Das Durchspülen der einzelnen Kanäle erfolgt aufeinanderfolgend, so daß im Falle einer fehlenden Durchgängigkeit der Durchflußwächter 34 ein entsprechendes Signal an die Elektronik 12 abgibt, die dann aufgrund des Programmablaufes sofort erkennt, welcher Kanal des Endoskopes beispielsweise durch Körpergewebe, Sekret oder dergleichen verlegt ist. Als Hinweis für die Bedienungsperson erscheint dann auf dem Display 17 beispeilsweise die Anzeige "I verstopft", wobei die Ziffer I für den ersten Kanal steht. Gleichzeitig leuchtet die Anzeige 35 auf und ein akustisches Signal ertönt. Nach erfolgter manueller Widerherstellung der Durchgängigkeit muß die Taste 19 erneut betätigt werden, um die weiteren Kanäle zu überprüfen.

Nach erfolgter Durchgängigkeitsüberprüfung erscheint auf dem Display 17 die Anzeige "Vorreinigung" und die digitale Zeitanzeige als Hinweis auf die Dauer der Vorreinigung mittels der in der Wanne 7 befindlichen Spülflüssigkeit, die, um anhaftende Teile an der Endoskopoberfläche zu entfernen, mittels der Pumpe 36, zur Erzeugung einer Strömung, ständig umgewälzt wird. Mit Ablauf der Vorreinigung erlischt die Anzeige 18 und die Anzeige 37 "Ablauf Wasser" leuchtet auf. Zusätzlich erscheint auf dem Display 17 die Anzeige "Hebel betätigen", was bedeutet, daß der Hebel 15 aus seiner Mittenposition nach rechts verschwenkt werden muß. Nach erfolgtem Umlegen des Hebels 15 erscheint dann auf dem Display 17 die Anzeige "Wasser läuft ab". Die Spülflüssigkeit gelangt hierbei aus der Wanne 7 über den Umschalthahn 15, der direkt mit dem Umschalthebel 15 betätigt wird, in den Aufnahmebehälter 9. Ist die Wanne 7 nach Ablauf einer bestimmten Zeit vollständig geleert, so erscheint auf dem Display 17 die Anzeige "ausblasen" sowie die hierzu vorgesehene Zeit in digitaler Form. Nach Ablauf der Ausblas-Zeit erscheint auf dem Display 17 die Anzeige "Betrieb". Gleichzeitig leuchtet das Anzeigenfeld 18 auf. Auf diese Aufforderung hin, ist der Hebel 15 wiederum in Mittenstellung zu bringen, was die Anzeige auf dem Display 17 auslöst "Desinfektionsmittel einfüllen", das dann manuell zu erfolgen hat. Nach dem Eingießen des Desinfektionsmittels durch die Öffnung 25 in der Abdeckung 8 ist die blinkende Taste 19 zu betätigen und mit der Taste 44 die erforderliche Desinfektionszeit vorzuwählen, wobei diese Zeit in dem Anzeigenfeld 39 durch Aufleuchten des eingestellten Wertes angezeigt wird. Im Anschluß daran bzw. für den Fall, daß eine bereits voreingestellte Zeitdauer für den Desinfektionsablauf übernommen wird, ist die Taste 19 erneut zu betätigen. Auf dem Display 17 erscheint nun die Anzeige "Desinfektion" und die eingestellte Zeit in digitaler Form.

Um, im Falle eines bereits durch mehrere Desinfektionen gefüllten Aufnahmebehälter 9, ein Überlaufen zu vermeiden, erscheint während des Desinfektionsvorganges auf dem Display wechselweise die Anzeige "Desinfektion" bzw. "Behälter leeren" als Hinweis für die Bedienungsperson, den Behälter 9 zu entleeren. Mit Ablauf der Desinfektionszeit erscheint wiederum auf dem Display 17 die Anzeige "Hebel betätigen" und das Anzeigenfeld 40 "Desinfektionsmittelablauf" leuchtet auf. Durch das manuell vorzunehmende Umschalten des Hebels 15 aus seiner Mittenposition nach links erfolgt das Ablaufen des Desinfektionsmittels in den Behälter 10, welches somit für weitere Desinfektionen wiederum zur Verwendung steht. Nach vollständiger Entleerung der Wanne 7 erfolgt auf dem Display 17 die Anzeige "ausblasen" sowie die hierzu vorgesehene Zeit in digitaler Form. Nach Ablauf der Ausblaszeit erscheint auf dem Display 17 die Anzeige "Hebel betätigen". Der Umschalthebel 15 ist somit wiederum in die Mittenposition zurückzustellen. Gleichzeitig mit dem Umschalten des Hebels 15 leuchtet das Anzeigenfeld 18 auf. Die nun wiederum blinkende Taste 19 ist zu betätigen, wodurch auf dem Display 17 die Anzeige "Sterilwasser einfüllen" ausgelöst wird. Das Einfüllen des Sterilwassers ist, wie bereits im Zusammenhang mit dem Einfüllen von Wasser vorstehend erwähnt, vorzunehmen. Im Anschluß an das Füllen der Wanne 7 mit Sterilwasser, ist die blinkende Taste 19 erneut zu betätigen, wodurch auf dem Display 17 die Anzeige "Neutralisation" sowie die hierfür vorgesehene Zeit in digitaler Form angezeigt wird. Nach Ablauf der Neutralisationszeit erscheint auf dem Display die Anzeige "Behälter leer?" und die Taste 19 blinkt. Auf diese Anweisung hin ist zu überprüfen, ob der Aufnahmebehälter 9 tatsächlich leer ist bzw. die in der Wanne 7 befindliche Flüssigkeit noch aufnehmen kann. Hat man sich hiervon überzeugt bzw. den Behälter geleert, so ist im Anschluß daran die Taste 19 erneut zu betätigen. Durch diese Betätigung erscheint auf dem Display 17 "Hebel betätigen" und das Anzeigenfeld 37 leuchtet auf. Auf diese Anweisung hin, ist der Hebel 15 von seiner Mittenposition wiederum nach rechts zu verschwenken, was bewirkt, daß über das Umschaltventil 15 das Sterilwasser in den Behälter 9 ablaufen kann. Ist der Behälter 7, nach Ablauf einer bestimmten Zeit, vollständig geleert, so erscheint auf dem Display 17 die Anzeige "ausblasen" sowie die hierfür vorgesehene Zeit in digitaler Form. Mit Ablauf der Ausblaszeit erlischt die Anzeige 18 und auf dem Display 17 erscheint die Anzeige "Programmende".

Neben dem vorstehend erläuterten Funktionsablauf besteht bei dem erfindungsgemäßen Desinfektor weiterhin die Möglichkeit, die Standzeit des Desinfektionsmittels zu überprüfen, wobei mittels der beiden Tasten 38 sowohl die Anzahl der maximal durchzuführenden Desinfektionszyklen als auch die vom Hersteller angegebene Verbrauchszeit in Tagen eingestellt werden kann. Die Anzahl der Desinfektionszyklen erscheint als digitaler Wert auf dem Anzeigenfeld 41, wobei während des Betriebes in Abhängigkeit der bereits durchgeführten Desinfektionen die noch verbleibenden Desinfektionszyklen abgelesen werden können. Die Standzeit der Desinfektionsflüssigkeit in Tagen kann auf dem Anzeigenfeld 42 abgelesen werden. In diesem Fall wird angezeigt, wieviele Tage das Desinfektionsmittel bereits verwendet wird. Ist nach Ablauf der vorgegebenen Standzeit ein Wechsel der Desinfektionsflüssigkeit erfolgt, so kann der mittels der Tasten 38 eingegebene Standzeitwert über die Taste 42 gelöscht bzw. der hierfür vorgesehene Speicher zurückgesetzt werden.

Der Vollständigkeit halber sei noch erwähnt, daß der Desinfektor auch während des Ladevorganges des Akkumulators betrieben werden kann, so daß ein kontinuierlicher Einsatz bzw. Betrieb des Desinfektionsgerätes möglich ist. Durch den halbautomatischen Desinfektionsablauf ist gewährleistet, daß einerseits die einzelnen Schritte nacheinanderfolgend ausgeführt und diese auch ständig überwacht werden.

In weiterer Ausbildung des erfindungsgemäßen Desinfektors besteht im Zusammenhang mit der Überwachung der Endoskopkanäle 1 bis 3 die Möglichkeit, anstelle des Durchflußwächters 34, jedem einzelnen Schlauchanschluß zwischen den Magnetventilen 31 bis 33 und den Anschlüssen 27 jeweils einen separaten Druckwächter vorzusehen, so daß ein gleichzeitiges Durchspülen und Überwachen aller drei Kanäle des Endoskopes erfolgen kann.

Zu erwähnen ist noch, daß mit dem Anzeigenfeld 45 die momentane Kapazität des Akkumulators überprüft werden kann. Des weiteren ist noch festzuhalten, daß das Aufleuchten des Tastenfeldes 46 auf den erforderlichen Wechsel des Schmutzfilters 30 hinweist. Nach erfolgtem Austausch dieses Schmutzfilters kann die Anzeige des Tastenfeldes 46 durch Betätigen desselben gelöscht werden.

**Ansprüche**

1. Verfahren zum Desinfizieren von flexiblen Endoskopen in einer mit Spül-, Desinfektions- und Neutralisationsflüssigkeit beschickbaren, in einem fahrbaren Gehäuse angeordneten Wanne, dadurch gekennzeichnet, daß für ein in die Wanne eingelegtes Endoskop ein Desinfektionsprogramm aus Reinigen des äußeren Endoskopschaftes mittels strömenden, manuell in die Wanne zu füllenden Spülwassers unter Zugabe eines flüssigen Spülmittels, aus Prüfung der Endoskopkanäle auf Durchgängigkeit mittels der im Kreislauf geführten Spülflüssigkeit, aus Ablauf der Spülflüssigkeit, aus Durchblasen der Kanäle, aus Einfüllen eines flüssigen Desinfektionsmittels in die Wanne mit deren Kreislauf durch die Kanäle, aus Ablauf des flüssigen Desinfektionsmittels mit nachfolgendem Durchblasen der Kanäle und aus Neutralisation des in den Kanälen befindlichen restlichen Desinfektionsmittels durch das ein Sterilfilter durchströmende Wasser mit anschließendem Durchblasen durchgeführt wird, wobei jedem Einzelschritt des Desinfektionsprogrammes eine entsprechende auf einem ablesbaren Anzeigenfeld in Klartext erscheinende Anzeige in Form einer Anweisung sowie ein visuelles und akustisches Signal vorausgeht, aufgrund dessen dann der von Hand auszulösende Desinfektionsvorgang des netzunabhängig zu betreibenden Desinfektors auszuführen ist.

2. Vorrichtung zur Durchführung des Verfahrens nach Anspruch 1, unter Verwendung einer ein Endoskop aufnehmenden, mit Spül-, Desinfektions- und Neutralisationsflüssigkeit beschickbaren Wanne in einem fahrbaren Gehäuse, dadurch gekennzeichnet, daß die Wanne (7) manuell aus einem Behälter (10) mit Desinfektionsflüssigkeit zur Desinfektion der Endoskopkanäle beschickbar ist, daß die Wanne aus einer Wasserleitung über ein Sterilfilter (6) zur Neutralisation des in den Endoskopkanälen befindlichen restlichen Desinfektionsmittels mit Sterilwasser beschickbar ist, daß der jeweilige Inhalt der Wanne im Kreislauf durch die Pumpe (28) nacheinander den Stutzen (27) zuführbar sind, an die die Kanäle des Endoskopes anschließbar sind und daß eine den Ablauf steuernde Elektronik (12,13) nach Einschalten der jeweils von Hand nacheinander einzuleitenden Vorgänge den Programmschritt mit Zeitangabe vorher anzeigt, bei Störungen des Desinfektionsprogrammes dieses unterbricht und die Störung sowohl im Klartext auf der Anzeige als auch durch Aufleuchten einer Anzeige sowie durch Ertönen eines akustischen Signals anzeigt.

3. Vorrichtung nach Anspruch 1 oder 2, dadurch gekennzeichnet, daß die an die Stutzen (27) anschließbaren Endoskopkanäle im Kreislauf aus der Wanne (7) über ein Schmutzfilter (30) und eine Pumpe nacheinander durch die Elektronik gesteuert über Magnetventile (31 bis 33) mit Spülwasser beschickbar sind und eine Verstopfung eines oder mehrerer Endoskopkanäle einen auf dem Anzeigenfeld erscheinenden Hinweis und sowohl ein optisches als auch akustisches Signal auslöst.

4. Vorrichtung nach Anspruch 1 bis 3, dadurch gekennzeichnet, daß für die Vorreinigung des Endoskopschaftes eine Umwälzpumpe (36) zum Umwälzen der Spülflüssigkeit in der Wanne vorgesehen ist, deren zeitlich festgelegtes Betriebsende ein Signal zur Handbetätigung eines Ventils (15) für den Ablauf der Spülflüssigkeit in einen im Gehäuse (2) vorgesehenen Aufnahmebehälter (9) auslöst.

5. Vorrichtung nach einem der Ansprüche 1 bis 4, dadurch gekennzeichnet, daß die Wanne (7) von einem tranparenten Deckel (8) abgedeckt ist, der mit zwei Durchbrüchen (9,25) versehen ist, die zur Zufuhr von Spülflüssigkeit und Reinigungsmitteln in die Wanne dienen.

FIG 4

FIG. 1

FIG. 2

FIG. 3

FIG. 5

EP 0 401 594 A2

FIG. 6

EP 0 401 594 A2

FIG. 7

EP 0 401 594 A2